(19) Europäisches Patentamt
European Patent Office
Office européen des brevets

(11) Publication number: **0 126 651**
**A2**

# (12) EUROPEAN PATENT APPLICATION

(21) Application number: 84303456.2

(22) Date of filing: 22.05.84

(51) Int. Cl.³: C 07 D 231/20, C 07 D 231/18, C 07 D 403/10, A 61 K 31/415

(30) Priority: 23.05.83 US 497316
12.03.84 US 586430

(43) Date of publication of application: 28.11.84 Bulletin 84/48

(84) Designated Contracting States: AT BE CH DE FR GB IT LI LU NL SE

(71) Applicant: WARNER-LAMBERT COMPANY, 201 Tabor Road, Morris Plains New Jersey 07950 (US)

(72) Inventor: Morrison, Glenn C., 1445 Arlington, Ann Arbor Michigan 48104 (US)

(74) Representative: Jones, Michael Raymond et al, HASELTINE LAKE & CO. 28 Southampton Buildings Chancery Lane, London WC2A 1AT (GB)

(54) **2,4-Dihydro-5-((substituted)phenyl)-4,4-disubstituted-3H-pyrazol-3-ones, 2,4-dihydro-5-((substituted)phenyl)-4, 4-disubstituted-3H-pyrazol-3-thiones, and a process for producing the same.**

(57) 2,4-Dihydro-5-[(substituted)phenyl]-4,4-disubstituted-3H-pyrazol-3-one and -3-thione compounds suitable for use as cardiotonic and antihypertensive agents are disclosed. A pharmaceutical composition and a process for the preparation of the said compounds are also described.

EP 0 126 651 A2

# 2,4-DIHYDRO-5-[(SUBSTITUTED)PHENYL]-4,4-DISUBSTITUTED-3H-PYRAZOL-3-ONES; 2,4-DIHYDRO-5-[(SUBSTITUTED)PHENYL]-4,4-DISUBSTITUTED-3H-PYRAZOL-3-THIONES; AND A PROCESS FOR PRODUCING THE SAME.

2,4-Dihydro-5-phenyl-4,4-dimethyl-3H-pyrazol-3-one is described in Bull. Soc. Chim. Fr., 1969, 4159 with no significant pharmacological activity prescribed.

The present invention relates to novel 2,4-dihydro-5-[(substituted)phenyl]-4,4-disubstituted-3H-pyrazol-3-ones and 2,4-dihydro-5-[(substituted)phenyl]-4,4-disubstituted-3H-pyrazol-3-thiones useful as cardiotonic and antihypertensive agents.

According to one aspect of the present invention, there is provided a compound having the following general formula I:

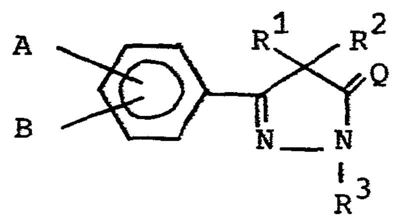

I

and pharmaceutically acceptable salts thereof, wherein:

Q is an oxygen or a sulphur atom;

$R^1$ is a $C_{1-6}$ lower alkyl radical; $R^2$ is a $C_{1-6}$ lower alkyl radical, a cyano group, an $S(O)_m R^1$ radical wherein m is 0, 1 or 2, an $-O-R^1$ radical or a triflurometh yl radical; or $R^1$ and $R^2$, when taken together, form a three to six membered carbon atom ring;

$R^3$ is a hydrogen atom or a $C_{1-6}$ lower alkyl radical; and

either each of A and B, which may be the same or different, is an alkoxy radical of from one to six carbon atoms, or B is a hydrogen atom and A, which is attached to the 3- or 4-position of the phenyl ring, is any one of the groups from (a) to (h) and (j) below:

(a)

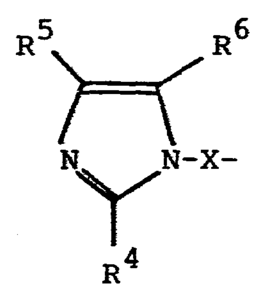

wherein:

$R^4$, $R^5$ and $R^6$ are independently chosen from a hydrogen atom; a $C_{1-6}$ lower alkyl group; a methylthio group; a methanesulphinyl group; a mesyl group; a hydroxylalkyl group, for instance a hydroxymethyl group; a halogen atom; and the radical $-(CH_2)_kNR^7R^8$, wherein k is 0, 1 or 2, and $R^7$ and $R^8$ are, independently, a hydrogen atom or a $C_{1-6}$ lower alkyl group; and $R^5$ and $R^6$ may further be chosen so that when taken together they form a (i) 5-, 6-, or 7-membered ring which may also contain a nitrogen atom, (ii) a benzene ring which is optionally substituted by one or more of the following: a halogen atom, a hydroxy radical, a $C_{1-6}$ lower alkyl radical, and a $C_{1-6}$ lower alkyloxy radical, or (iii) a pyridine ring;

X is a bond, a $-(CH_2)_n-$ group or a $-O(CH_2)_{n+1}-$ group wherein n is an integer of from one to four;

(b)

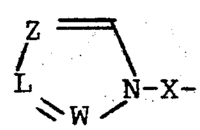

wherein

(i) W = L = Z = CH,

(ii) W = Z = N and L = CH, or

(iii) L = Z = N and W = CH; and

X is the same as defined in (a) above;

(c)

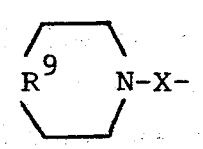

wherein $R^9$ is a methylene group, an oxygen atom, a sulphur atom or the radical $\geq NR^{10}$, wherein $R^{10}$ is a hydrogen atom; a $C_{1-6}$ lower alkyl group; the radical $-COR^{11}$, wherein $R^{11}$ is a benzene ring optionally substituted by one or more of the following: a halogen atom, a $C_{1-6}$ lower alkyl radical, a hydroxy radical, a $C_{1-6}$ lower alkoxy group, and a $CF_3$ group; or $R^{10}$ is the radical $-(CH_2)_pR^{11}$, wherein p is zero or an integer of from one to four and $R^{11}$ is the same as defined above; and

X is the same as defined in (a) above;

(d)

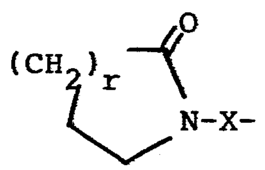

wherein r is from one to three; and

X is the same as defined in (a) above;

(e)

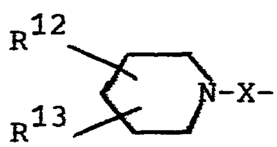

wherein $R^{12}$ and $R^{13}$ are independently chosen from the following: a hydrogen atom; a $C_{1-6}$ lower alkyl radical; an aryl radical; a hydroxy radical; a $C_{1-6}$ lower alkoxy radical; $-OCOR^{14}$ or $-OCONHR^{14}$, wherein $R^{14}$ is a $C_{1-6}$ lower alkyl radical, an aryl radical or a heteroaryl radical or, when taken together, $R^{12}$ and $R^{13}$ constitute a carbonyl group or an ethylenedioxy group; and

X is the same as defined in (a) above

(f)

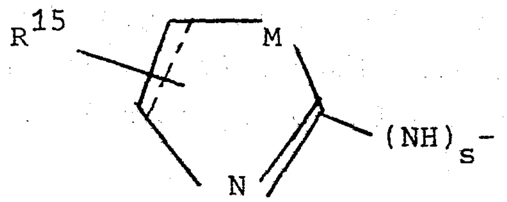

wherein ======= represents a double or single bond between two carbon atoms; $R^{15}$ is a hydrogen atom or a $C_{1-6}$ lower alkyl radical; M is $>$NH, or an oxygen atom or a sulphur atom; and s is 0 or 1;

(g)

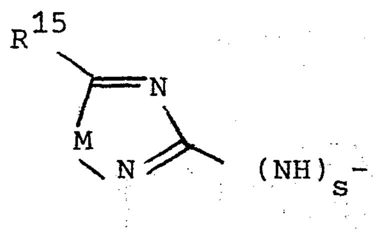

wherein $R^{15}$, M and s are the same as defined in (f) above;

(h) $-NHPR^{16}R^{17}$ wherein P is a bond or a carbonyl group; $R^{16}$ is a $C_{1-6}$ lower alkylene group which may be straight or branched; $R^{17}$ is a hydrogen atom, a $C_{1-6}$ lower alkyl, a halogen atom, the radical $-NR^{18}R^{19}$, wherein $R^{18}$ and $R^{19}$, which may be the same or different, are chosen from a hydrogen atom, a $C_{1-6}$ lower alkyl radical which is straight or branched, a group as defined in (a) - (e) above, or, when taken together, with the nitrogen atom to which they are attached, $R^{18}$ and $R^{19}$ form a 5-, 6-, or 7-membered ring or a group as defined in (a) - (e) above wherein X is a bond; or $R^{17}$ is the radical $-S(O)_vR^{20}$ where v is 0, 1 or 2 and $R^{20}$ is a $C_{1-6}$ lower alkyl group which may be straight or branched, or a phenyl radical;

(j) $-S(O)_vR^{20}$ where v is 0, 1 or 2 and $R^{20}$ is a $C_{1-6}$ lower alkyl group which may be straight or branched or a phenyl radical.

The compounds of formula I where $R^3$ is a hydrogen atom and Q is an oxygen atom may exist in tautomeric forms, for example, as illustrated by the following formulae.

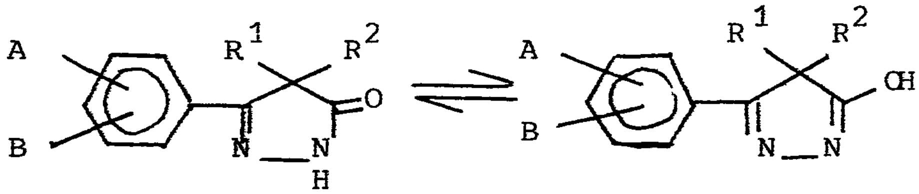

The present invention also relates to a compound of the formula

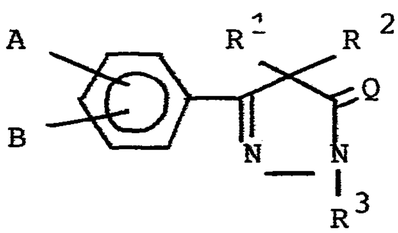

wherein $R^1$, $R^2$, $R^3$ and X are as defined above and where A and B are each an alkoxy radical of from one to six carbon atoms, preferably substituted in the 3- and 4-positions. Particularly preferred are compounds in which A and B are both methoxy.

The present invention also relates to a compound having the following general formula:

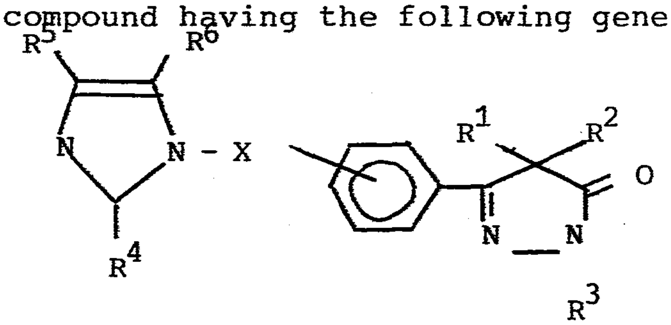

and pharmaceutically acceptable salts thereof, wherein $R^1$, $R^2$, $R^3$, $R^4$, $R^5$, $R^6$, and X are as defined above.

According to another aspect of the present invention, there is provided a compound having the following general formula:

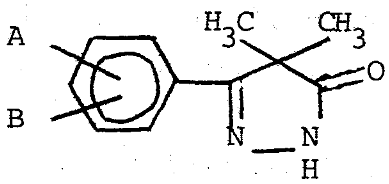

and pharmaceutically acceptable salts thereof, wherein each of A and B is an alkoxy radical of from one to six carbon atoms, or where B is a hydrogen atom and A is as defined in parts (a) to (h) and (j) above. Preferred groups for A are an imidazole radical or an imidazole radical substituted by pharmaceutically acceptable salts thereof, wherein B is a hydrogen atom and A is one of the following: an imidazole radical; an imidazole radical substituted by one or more of the following: a $C_{1-6}$ lower alkyl radical, a $C_{1-6}$ S-lower alkyl radical, and a $-CH_2OH$ radical; a tetrahydrobenzimidazole radical; a benzimidazole radical; a 1,2,4-triazole radical; a 2-thiazoline radical; a 4-hydroxy- piperidine radical; a 1,4-dioxa-8-azaspiro[4,5] decane radical; or a N-methylpiperazine radical.

Particularly preferred compounds of the present invention include:

2,4-Dihydro-5-[4-(1<u>H</u>-imidazol-1-yl)phenyl]-4,4-dimethyl-3<u>H</u>-pyrazol-3-one and pharmaceutically acceptable acid addition salts thereof;

4-Ethyl-2,4-dihydro-5-[4-(1<u>H</u>-imidazol-1-yl)phenyl]-4-methyl-3<u>H</u>-pyrazol-3-one and pharmaceutically acceptable acid addition salts

thereof;

2,4-Dihydro-5-[4-(1H-benzimidazol-1-yl)phenyl]-4,4-dimethyl-3H-pyrazol-3-one and pharmaceutically acceptable acid addition salts thereof;

2,4-Dihydro-5-[4-(4,5,6,7-tetrahydro-1H-benzimidazol-1-yl-phenyl]-4,4-dimethyl-3H-pyrazol-3-one and pharmaceutically acceptable acid addition salts thereof;

2,4-Dihydro-5-[4-(1,4-dioxa-8-azaspiro[4,5]dec-8-yl)phenyl]-4,4-dimethyl-3H-pyrazol-3-one and pharmaceutically acceptable acid addition salts thereof;

2,4-Dihydro-5-[4-(1H-1,2,4-triazol-1-yl]phenyl]-4,4-dimethyl-3H-pyrazol-3-one and pharmaceutically acceptable acid addition salts thereof; and

2,4-Dihydro-5-(3,4-dimethoxyphenyl)-4,4-dimethyl-3H-pyrazol-3-one and pharmaceutically acceptable acid addition salts thereof.

According to a further aspect of the present invention there is provided a pharmaceutical composition comprising an effective amount of a compound as described above in admixture with a pharmaceutically acceptable carrier.

The process for producing the pyrazol-3-ones of the formula I comprises reacting an appropriately substituted $\alpha,\alpha$-disubstituted-$\beta$-oxobenzenepropionic acid ester of the following general formula:

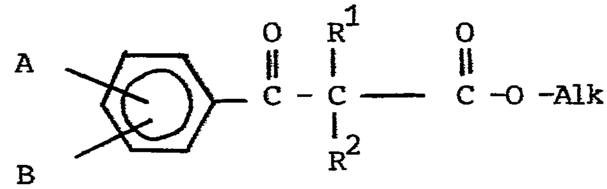

with a hydrazine of the formula $R^3NHNH_2$, wherein A, B, $R^1$, $R^2$ and $R^3$ are as defined above and Alk is an alkyl radical of from 1 to 4 carbon atoms, preferably a methyl or an ethyl radical, at the boiling point of the solvent, which, for example, may be an alcohol such as methanol or ethanol.

The compounds of the formula I where Q is a sulphur atom may conveniently be prepared from the above products for which Q is an oxygen atom by conventional means, such as treating the oxo compounds with phosphorus pentasulphide.

The starting acid esters may be prepared by alkylating, in one step or in a step-wise manner depending on whether $R^1$ and $R^2$ are the same or different, the appropriately substituted alkyl ester of $\beta$-oxobenzenepropionic acid with an alkyl halide in the presence of sodium hydride in a polar aprotic solvent, such as dimethylformamide. The halide of preference is the iodide. The preferred ester is an alkyl ester of from one to four carbon atoms, most preferably the methyl ester or ethyl ester.

The compounds of formula (I) are useful both in the free base form and in the form of an acid addition salt. Both forms are within the scope of the invention. The acid addition salts are a more convenient form to use; in practice, use of the salt form amounts to use of the base form. In practicing the invention, it was found convenient to form the sulphate, phosphate, or methanesulphonate salts. However, other appropriate pharmaceutically acceptable salts within the scope of the invention are those derived from mineral acids, for example, hydrochloric acid and sulphamic acid; and organic acids, for example ethanesulphonic acid,

benzenesulphonic acid, p-toluene-sulphonic acid; giving the hydrochloride, sulphamate, ethanesulphonate, benzenesulphonate, p-toluene-sulphonic respectively.

The acid addition salts of the basic compounds are prepared either by dissolving the free base in aqueous, or aqueous alcohol, solution or other suitable solvents containing the appropriate acid and isolating the salt by evaporating the solution, or by reacting the free base and acid in an organic solvent, in which case the salt separates directly or can be obtained by concentration of the solution.

The term "lower" in reference to an alkyl radical and an alkoxy radical means a straight or branched hydrocarbon chain of from one to six carbon atoms, for example, methyl, ethyl, propyl, isopropyl. The term "halogen" includes fluorine, chlorine, bromine, and iodine but preferably is fluorine or chlorine.

The following examples will further illustrate the invention without, however, limiting thereto.

EXAMPLE I

<u>1-(Substituted phenyl)ethanones</u>

A mixture of 0.2 moles of 4-fluoroacetophenone, 0.2 moles of an amine, 0.41 moles of potassium carbonate, 0.025 moles of cupric oxide and 80 ml of pyridine is refluxed under a nitrogen atmosphere for 24 to 48 hours. The pyridine is removed in vacuo and the residue is treated with chloroform and water. The chloroform layer is passed through silica gel and the solvent is removed. The residue is crystallized from methylene chloride-isopropyl ether.

According to the above method, the following compounds were obtained:

1-[4-(1H-Benzimidazol-1-yl)phenyl]ethanone as a crystalline solid, mp 134-135°C.

1-[4-(4,5,6,7-Tetrahydro-1H-benzimidazol-1-yl)phenyl] ethanone as a crystalline solid, mp 94-98°C.

1-[4-(1,4-Dioxa-8-azaspiro[4,5]dec-8-yl)phenyl] ethanone as a crystalline solid, mp 120-121°C.

EXAMPLE 2

Substituted-β-oxobenzenepropanoic acid methyl esters

To a mixture of 0.1 mole of sodium hydride (60% suspension in Nujol), 12 ml of dimethyl carbonate and 15 ml of tetrahydrofuran is added dropwise a solution of a 0.05 moles of a 1-(substituted phenyl) ethanone in dimethylformamide-tetrahydrofuran. The mixture is heated at reflux for three hours. The reaction mixture is poured into water, the pH is adjusted to 9, and the mixture is extracted with methylene chloride. The solvent is removed and the residue crystallized from methylene chloride-isopropyl ether.

By the above method, the following compounds were obtained:

4-(1H-Benzimidazol-1-yl)-β-oxobenzenepropionic acid methyl ester as a crystalline solid, mp 130-133°C.

4-(4,5,6,7-Tetrahydro-1H-benzimidazol-1-yl)-β-oxobenzenepropanoic acid methyl ester as a crystalline solid, mp 131-133°C.

4-(1,4-Dioxa-8-azaspiro[4,5]dec-8-yl)-β-oxobenzenepropanoic acid methyl ester as a crystalline solid, mp 85-88°C.

4-(1H-1,2,4-Triazol-1-yl)-β-oxobenzenepropanoic acid methyl ester as a crystalline solid, mp 123-125°C.

3,4,-Dimethoxy-β-oxobenzenepropanoic acid methyl ester as an oil, bp 110°C (0.4 mm Hg).

## EXAMPLE 3

Substituted-α,α-dimethyl-β-oxobenzenepropanoic acid methyl esters

To a slurry of 0.2 moles of sodium hydride (60% dispersion in Nujol) in 30 ml of dimethylformamide is added a solution of 0.1 moles of a substituted-β-oxobenzenepropanoic acid methyl ester in 100 ml of dimethylformamide at 15-20°C. After the addition is completed, a solution of 0.1 moles of methyl iodide is added and stirring is continued for 20 hours at room temperature. The reaction mixture is poured into 1.2 ℓ of water and is extracted with ether. The ether is removed and the reside triturated with petroleum ether.

Accordingly, the following compounds were obtained:

4-(1H-imidazol-1-yl)-α,α-dimethyl-β-oxobenzenepropanoic acid methyl ester as a crystalline solid, mp 91-92°C after recrystallization from isopropyl ether.

4-(1H-Benzimidazol-1-yl)-α,α-dimethyl-β-oxobenzenepropanoic acid methyl ester as an oil.

4-(4,5,6,7-Tetrahydro-1H-benzimidazol-1-yl)-α,α-dimethyl-β-oxobenzenepropanoic acid methyl ester as an oil.

4-(1,4-Dioxa-8-azaspiro[4,5]dec-8-yl)-α,α-dimethyl-β-oxobenzenepropanoic acid methyl ester as an oil.

4-(1H-1,2,4-Triazol-1-yl)-α,α-dimethyl-β-oxobenzenepropanoic acid methyl ester as an oil.

3,4-Dimethoxy-α, α-dimethyl-β-oxobenzenepropanoic acid methyl ester as a crystalline solid, mp 84-85°C.

## EXAMPLE 4

α-Ethyl-4-(1H-imidazol-1-yl)-α-methyl-β-oxobenzenepropanoic acid methyl ester

To a slurry of 2.49 g of sodium hydride (60% dispersion in Nujol) in 50 ml of dimethylformamide

is added a solution of 24.4 g of 4-(1H-imidazol-1-yl)-β-oxobenzenepropanoic acid methyl ester in 75 ml of dimethylformamide at 15-20°C. After the addition is completed 15.6 g of ethyl iodide is added and stirring is continued for 48 hours. The reaction mixture is filtered, poured into water, and the mixture is extracted with ether. Removal of the solvent and trituration with isopropyl ether gives an oil. A solution of 2.72 g of the oil in 10 ml of dimethylformamide is added to a slurry of 0.24 g of sodium hydride (60% dispersion in Nujol) at 15-20°. A solution of 1.42 g of methyl iodide in 5 ml of dimethylformamide is added and stirring is continued for 20 hours at room temperature. The reaction mixture is filtered, poured into water, and is extracted with ether.

Removal of the solvent gives an oil. The hydrochloride may be obtained from ethanol-isopropyl ether as a crystalline solid, mp 164-166°C.

## EXAMPLE 5

<u>2,4-Dihydro-5-[substituted phenyl]-4,4-dimethyl-3H-pyrazol-3-ones</u>

A solution of 1.0 g of a substituted -α,α-dimethyl-β-oxobenzenepropanoic acid methyl ester and 0.5 g of hydrazine hydrate in 10 ml of ethanol is refluxed overnight. On cooling, a crystalline product is deposited.

Accordingly, the following compounds are obtained:

2,4-Dihydro-5-[4-(1H-imidazol-1-yl)phenyl]-4,4-dimethyl-3H-pyrazol-3-one as a crystalline solid, mp 170-171°C.

2,4-Dihydro-5-[4-(1H-benzimidazol-1-yl)phenyl]-4,4-dimethyl-3H-pyridazol-3-one as a crystalline solid, mp 301-304°C.

2,4-Dihydro-5-[4-(4,5,6,7-tetrahydro-1H-benzimidazol-1-yl)phenyl]-4,4-dimethyl-3H-pyrazol-3-one as a

crystalline solid, mp 245-246°C.

2,4-Dihydro-5-[4-(1,4-dioxa-8-azaspiro[4,5]dec-8-yl)phenyl]-4,4-dimethyl-3H-pyrazol-3-one as a crystalline solid, mp 163.5-165.5°C.

2,4-Dihydro-5-[4-(1H-1,2,4-triazol-1-yl)phenyl]-4,4-dimethyl-3H-pyrazol-3-one as a crystalline solid, mp 158-160°C.

2,4-Dihydro-5-(3,4-dimethoxyphenyl)-4,4-dimethyl-3*H*-pyrazol-3-one as a crystalline solid, mp 193-194°C.

EXAMPLE 6

4-Ethyl-2,4-dihydro-5-[4-(1H-imidazol-1-yl)phenyl]-4-methyl-3H-pyrazol-3-one

A solution of 3.8 g of α-ethyl-4-(1H-imidazol-1-yl)-α-methyl-β-oxobenzenepropanoic acid methyl ester, 2.0 g of hydrazine hydrate and 25 ml of ethanol was refluxed for 20 hours. The ethanol is removed and the residue is treated with ether and water. Filtration of the solid gives after recrystallization from ethyl acetate, 2.7 g of a crystalline solid, mp 86-88°C.

The usefulness of the compounds of the present invention as cardiotonic agents is demonstrated by their effectiveness in standard pharmacological test procedures, for example, in causing a significant increase in the myocardial contractility in the pentobarbital-anesthetized dog with low or minimal changes in heart rate and a moderate reduction in blood pressure. This test procedure is described in the following paragraphs.

Test for In Vivo Myocardial Intropic Activity in Anesthetized Dog

This screen consists of determining the effects of increasing intravenous doses of compound on mycardial contractility (dP/dt max of left ventricular blood pressure), heart rate, and aortic blood pressure of the pentobarbital-anesthetized dog.

Methods

Adult mongrel dogs of either sex are anesthetized with pentobarbital, 35 mg/kg, IV, and are subsequently maintained under anesthesia with a continuous infusion of pentobarbital, 3.5 mg/kg/hour. The trachea is intubated but the animals are permitted to breathe spontaneously. A cannula is inserted into the femoral vein for administrating test agents. A Millar catheter tip pressure transducer or a fluid filled catheter is inserted into the ascending aorta via the femoral artery for measuring aortic blood pressure. A Millar catheter tip pressure transducer is passed into the left ventricle via the left carotid artery for measuring left ventricular blood pressure. Needle electrodes are placed subcutaneously for recording a lead II electrocardiogram (ECG).

Left ventricular and aortic blood pressures are recorded on a strip chart recorder. Heart rate, using a biotachometer triggered from the R wave of the ECG, and the first derivative of left ventricular blood pressure (dP/dt), obtained with a differentiator amplifier coupled to the corresponding pressure amplifier, are also recorded. A period of at least 30 minutes is utilized to obtain control data prior to administration of test compound.

Depending on solubility, the compounds are dissolved in 0.9% saline solution or in dilute HCl or NaOH (0.1 or 1.0 N) and are diluted to volume with normal saline. Ethanol or dimethylacetamide can be used as solvents if adequate dilutions can be made. Appropriate vehicle controls are administered when needed.

Each dose of the test compound is administered in a volume of 0.1 ml/kg over a period of one minute.

When tested by the above-described Anesthetized Dog Procedure, the compounds of the present invention,

for example, 2,4-dihydro-5-[4-(1H-imidazol-1-yl)phenyl]-4,4-dimethyl-3H-pyrazol-3-one, when administered intravenously at a rate of about 0.01 to 0.31 mg/kg/min cause dose related significant increases in cardiac contractility with only low or minimal changes in heart rate and moderate lowering of blood pressure. Accordingly, the compounds of the present invention are also useful as antihypertensive agents.

CLAIMS (for the Contracting States BE, CH, DE, FR, GB, IT, NL, LI, LU, SE):

1. A compound having the following general formula I:

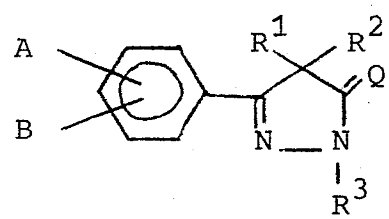

I

and pharmaceutically acceptable salts thereof, wherein:

Q is an oxygen or a sulphur atom;

$R^1$ is a $C_{1-6}$ lower alkyl radical; $R^2$ is a $C_{1-6}$ lower alkyl radical, a cyano group, an $S(O)_m R^1$ radical wherein m is 0, 1 or 2, an $-O-R^1$ radical or a trifluromethyl radical; or $R^1$ and $R^2$, when taken together, form a three to six membered carbon atom ring;

$R^3$ is a hydrogen atom or a $C_{1-6}$ lower alkyl radical; and

either each of A and B, which may be the same or different, is an alkoxy radical of from one to six carbon atoms, or B is a hydrogen atom and A, which is attached to the 3- or 4-position of the phenyl ring, is any one of the groups from (a) to (h) and (j) below:

(a)

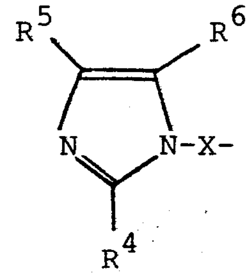

wherein:

$R^4$, $R^5$ and $R^6$ are independently chosen from: a hydrogen atom; a $C_{1-6}$ lower alkyl group; a methylthio group; a methanesulphinyl group; a mesyl group; a hydroxylalkyl group, for instance a hydroxymethyl group; a halogen atom; and the radical $-(CH_2)_kNR^7R^8$, wherein k is 0, 1 or 2, and $R^7$ and $R^8$ are, independently, a hydrogen atom or a $C_{1-6}$ lower alkyl group; and $R^5$ and $R^6$ may further be chosen so that when taken together they form a (i) 5-, 6-, or 7-membered ring which may also contain a nitrogen atom, (ii) a benzene ring which is optionally substituted by one or more of the following: a halogen atom, a hydroxy radical, a $C_{1-6}$ lower alkyl radical, and a $C_{1-6}$ lower alkyloxy radical, or (iii) a pyridine ring;

X is a bond, a $-(CH_2)_n-$ group or a $-O(CH_2)_{n+1}-$ group wherein n is an integer of from one to four;

(b)

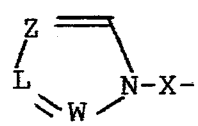

wherein

(i) $W = L = Z = CH$,

(ii) $W = Z = N$ and $L = CH$, or

(iii) $L = Z = N$ and $W = CH$; and

X is the same as defined in (a) above;

(c)

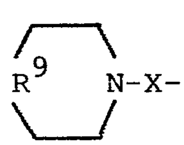

wherein $R^9$ is a methylene group, an oxygen atom, a sulphur atom or the radical $\geq NR^{10}$, wherein $R^{10}$ is a hydrogen atom; a $C_{1-6}$ lower alkyl group; the radical $-COR^{11}$, wherein $R^{11}$ is a benzene ring optionally substituted by one or more of the following: a halogen atom, a $C_{1-6}$ lower alkyl radical, a hydroxy radical, a $C_{1-6}$ lower alkoxy group, and a $CF_3$ group; or $R^{10}$ is the radical $-(CH_2)_pR^{11}$, wherein p is zero or an integer of from one to four and $R^{11}$ is the same as defined above; and

X is the same as defined in (a) above;

(d)

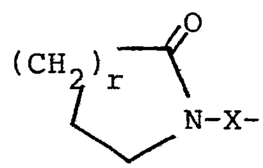

wherein r is from one to three; and

X is the same as defined in (a) above;

(e)

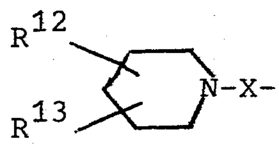

wherein $R^{12}$ and $R^{13}$ are independently chosen from the following: a hydrogen atom; a $C_{1-6}$ lower alkyl radical; an aryl radical; a hydroxy radical; a $C_{1-6}$ lower alkoxy radical; $-OCOR^{14}$ or $-OCONHR^{14}$, wherein $R^{14}$ is a $C_{1-6}$ lower alkyl radical, an aryl radical or a heteroaryl radical or, when taken together, $R^{12}$ and $R^{13}$ constitute a carbonyl group or an ethylenedioxy group; and

X is the same as defined in (a) above

(f)

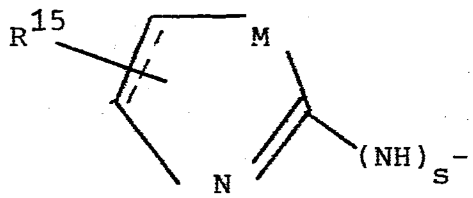

wherein $\overline{-------}$ represents a double or single bond between two carbon atoms; $R^{15}$ is a hydrogen atom or a $C_{1-6}$ lower alkyl radical; M is $>$NH, or an oxygen atom or a sulphur atom; and s is 0 or 1;

(g)

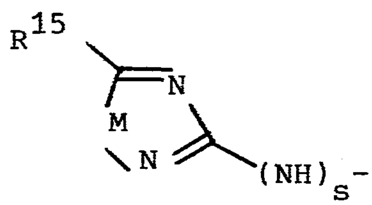

wherein $R^{15}$, M and s are the same as defined in (f) above;

(h) $-NHPR^{16}R^{17}$ wherein P is a bond or a carbonyl group; $R^{16}$ is a $C_{1-6}$ lower alkylene group which may be straight or branched; $R^{17}$ is a hydrogen atom, a $C_{1-6}$ lower alkyl, a halogen atom, the radical $-NR^{18}R^{19}$, wherein $R^{18}$ and $R^{19}$, which may be the same or different, are chosen from a hydrogen atom, a $C_{1-6}$ lower alkyl radical which is straight or branched, a group as defined in (a) - (e) above, or, when taken together, with the nitrogen atom to which they are attached, $R^{18}$ and $R^{19}$ form a 5-, 6-, or 7-membered ring or a group as defined in (a) - (e) above wherein X is a bond; or $R^{17}$ is the radical $-S(O)_vR^{20}$ where v is 0, 1 or 2 and $R^{20}$ is a $C_{1-6}$ lower alkyl group which may be straight or branched, or a phenyl radical;

(j) $-S(O)_vR^{20}$ where v is 0, 1 or 2 and $R^{20}$ is a $C_{1-6}$ lower alkyl group which may be straight or branched or a phenyl radical.

2. A compound according to Claim 1, having the following general formula:

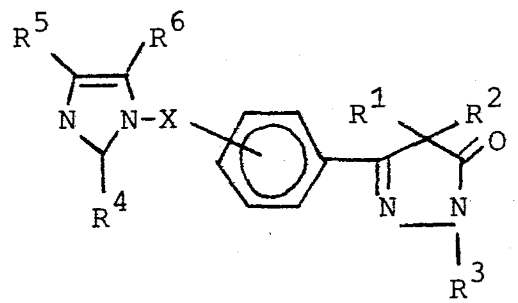

and pharmaceutically acceptable salts thereof, wherein $R^1$, $R^2$, $R^3$, $R^4$, $R^5$, $R^6$ and X are as defined in Claim 1.

3. A compound according to Claim 1, having the following general formula:

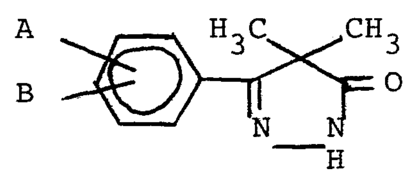

and pharmaceutically acceptable salts thereof, wherein A and B, which may be the same or different, are each an alkoxy radical of from one to six carbon atoms.

4. A compound according to Claim 1 or 2, having the following general formula:

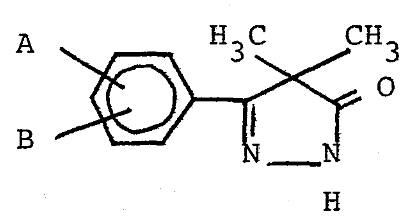

and pharamceutically acceptable salts thereof, wherein B is a hydrogen atom and A is as defined in parts (a) to (h) and (j) above in Claim 1.

5. A compound according to Claim 1, or to Claim 4, when appendant to Claim 1, and pharmaceutically acceptable salts thereof, wherein

B is a hydrogen atom and A is one of the following: an imidazole radical; an imidazole radical substituted by one or more of the following: a $C_{1-6}$ lower alkyl radical, a $C_{1-6}$ S-lower alkyl radical, and a $-CH_2OH$ radical; a tetrahydrobenzimidazole radical; a benzimidazole radical; a 1,2,4-triazole radical; a 2-thiazoline radical; a 4-hydroxy-piperidine radical; a 1,4-dioxa-8-azaspiro[4,5] decane radical; or a N-methylpiperazine radical.

6. A compound according to Claim 5, having one of the following names:
2,4-dihydro-5-[4-(1H-imidazol-1-yl)phenyl]-4,4-dimethyl-3H-pyrazol-3-one;
2,4-dihydro-5-[4-(1H-benzimidazol-1-yl)phenyl]-4,4-dimethyl-3H-pyrazol-3-one;
2,4-dihydro-5-[4-(4,5,6,7-tetrahydro-1H-benzimidazol-1-yl)phenyl]-4,4-dimethyl-3H-pyrazol-3-one;
2,4-dihydro-5[4-(1,4-dioxa-8-azaspiro[4,5]dec-8-yl)phenyl]-4,4-dimethyl-3H-pyrazol-3-one; and
2,4-dihydro-5-[4-(1H-1,2,4-triazol-1-yl)phenyl]-4,4-dimethyl-3H-pyrazol-3-one; or a pharmaceutically acceptable salt thereof.

7. A compound according to Claim 3, having the name:
2,4-dihydro-5-(3,4-dimethoxyphenyl)-4,4-dimethyl-3H-pyrazol-3-one; or a pharmaceutically acceptable salt thereof.

8. A compound according to Claim 1 or 2, having the name:
4-ethyl-2,4-dihydro-5-[4-(1H-imidazol-1-yl)phenyl]-4-methyl-3H-pyrazol-3-one; or a pharmaceutically acceptable salt thereof.

9. A pharmaceutical composition comprising an effective amount of a compound as claimed in any preceding claim, in admixture with a pharmaceutically acceptable carrier.

10. A process for the preparation of a compound as claimed in any one of Claims 1 to 8, which process comprises reacting a compound of the following general formula:

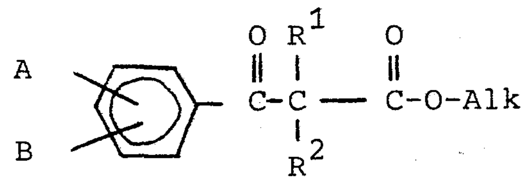

with a hydrazine of the formula $R^3NHNH_2$ wherein Alk is an alkyl radical of from 1 to 4 carbon atoms and A, B, $R^1$, $R^2$ and $R^3$ are as defined in Claim 1, at the boiling point of the solvent, and, if desired, converting the resulting product to a compound as claimed in Claim 1 wherein Q is a sulphur atom by treatment with phosphorus pentasulphide.

CLAIMS (for the Contracting State AT)

1. A process for the preparation of a compound of the following general formula I:

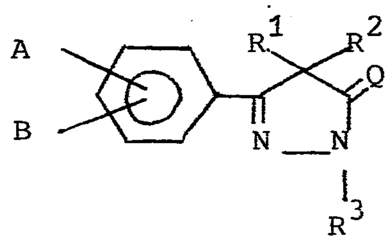

I

and pharmaceutically acceptable salts thereof, wherein:

Q is an oxygen or a sulphur atom;

$R^1$ is a $C_{1-6}$ lower alkyl radical; $R^2$ is a $C_{1-6}$ lower alkyl radical, a cyano group, an $S(O)_m$ $R^1$ radical wherein m is 0, 1 or 2, an $-O-R^1$ radical or a trifluromethyl radical; or $R^1$ and $R^2$, when taken together, form a three to six membered carbon atom ring;

$R^3$ is a hydrogen atom or a $C_{1-6}$ lower alkyl radical; and

either each of A and B, which may be the same or different, is an alkoxy radical of from one to six carbon atoms, or B is a hydrogen atom and A, which is attached to the 3- or 4-position of the phenyl ring, is any one of the groups from (a) to (h) and (j) below:

(a)

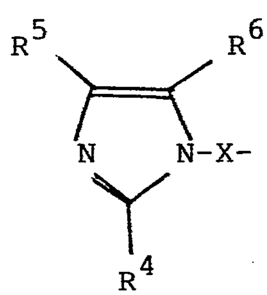

wherein:

$R^4$, $R^5$ and $R^6$ are independently chosen from a hydrogen atom; a $C_{1-6}$ lower alkyl group; a methylthio group; a methanesulphinyl group; a mesyl group; a hydroxylalkyl group, for instance a hydroxymethyl group; a halogen atom; and the radical $-(CH_2)_kNR^7R^8$, wherein k is 0, 1 or 2, and $R^7$ and $R^8$ are, independently, a hydrogen atom or a $C_{1-6}$ lower alkyl group; and $R^5$ and $R^6$ may further be chosen so that when taken together they form a (i) 5-, 6-, or 7-membered ring which may also contain a nitrogen atom, (ii) a benzene ring which is optionally substituted by one or more of the following: a halogen atom, a hydroxy radical, a $C_{1-6}$ lower alkyl radical, and a $C_{1-6}$ lower alkyloxy radical, or (iii) a pyridine ring;

X is a bond, a $-(CH_2)_n-$ group or a $-O(CH_2)_{n+1}-$ group wherein n is an integer of from one to four;

(b)

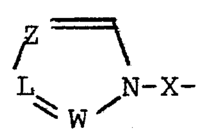

wherein

(i) W = L = Z = CH,

(ii) W = Z = N and L = CH, or

(iii) L = Z = N and W = CH; and

X is the same as defined in (a) above;

(c)

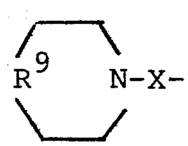

wherein $R^9$ is a methylene group, an oxygen atom, a sulphur atom or the radical $>NR^{10}$, wherein $R^{10}$ is a hydrogen atom; a $C_{1-6}$ lower alkyl group; the radical $-COR^{11}$, wherein $R^{11}$ is a benzene ring optionally substituted by one or more of the following: a halogen atom, a $C_{1-6}$ lower alkyl radical, a hydroxy radical, a $C_{1-6}$ lower alkoxy group, and a $CF_3$ group; or $R^{10}$ is the radical $-(CH_2)_pR^{11}$, wherein p is zero or an integer of from one to four and $R^{11}$ is the same as defined above; and

X is the same as defined in (a) above;

(d)

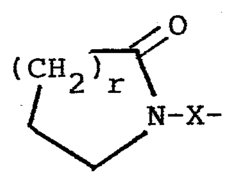

wherein r is from one to three; and

X is the same as defined in (a) above;

(e)

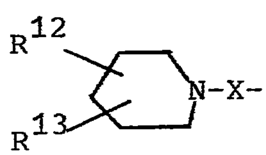

wherein $R^{12}$ and $R^{13}$ are independently chosen from the following: a hydrogen atom; a $C_{1-6}$ lower alkyl radical; an aryl radical; a hydroxy radical; a $C_{1-6}$ lower alkoxy radical; $-OCOR^{14}$ or $-OCONHR^{14}$, wherein $R^{14}$ is a $C_{1-6}$ lower alkyl radical, an aryl radical or a heteroaryl radical or, when taken together, $R^{12}$ and $R^{13}$ constitute a carbonyl group or an ethylenedioxy group; and

X is the same as defined in (a) above

(f)

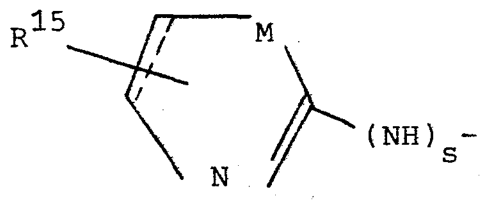

wherein ======= represents a double or single bond between two carbon atoms; $R^{15}$ is a hydrogen atom or a $C_{1-6}$ lower alkyl radical; M is >NH, or an oxygen atom or a sulphur atom; and s is 0 or 1;.

(g)

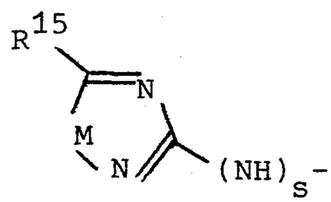

wherein $R^{15}$, M and s are the same as defined in (f) above;

(h) $-NHPR^{16}R^{17}$ wherein P is a bond or a carbonyl group; $R^{16}$ is a $C_{1-6}$ lower alkylene group which may be straight or branched; $R^{17}$ is a hydrogen atom, a $C_{1-6}$ lower alkyl, a halogen atom, the radical $-NR^{18}R^{19}$, wherein $R^{18}$ and $R^{19}$, which may be the same or different, are chosen from a hydrogen atom, a $C_{1-6}$ lower alkyl radical which is straight or branched, a group as defined in (a) - (e) above, or, when taken together, with the nitrogen atom to which they are attached, $R^{18}$ and $R^{19}$ form a 5-, 6-, or 7-membered ring or a group as defined in (a) - (e) above wherein X is a bond; or $R^{17}$ is the radical $-S(O)_vR^{20}$ where v is 0, 1 or 2 and $R^{20}$ is a $C_{1-6}$ lower alkyl group which may be straight or branched, or a phenyl radical;

(j) $-S(O)_vR^{20}$ where v is 0, 1 or 2 and $R^{20}$ is a $C_{1-6}$ lower alkyl group which may be straight or branched or a phenyl radical.

which process comprises reacting a compound of the following general formula:

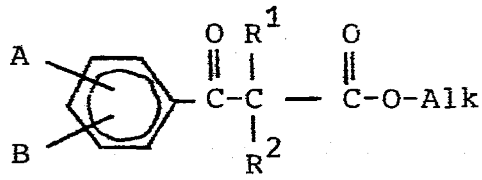

with a hydrazine of the formula $R^3NHNH_2$, wherein Alk is an alkyl radical of from 1 to 4 carbon atoms and A, B, $R^1$, $R^2$ and $R^3$ are as defined above, at the boiling point of the solvent, and, if desired, converting the resulting product to a compound as claimed in Claim 1 wherein Q is a sulphur atom by treatment with phosphorus pentasulphide; and optionally converting the resulting compound to a pharmaceutically acceptable salt.

2. A process according to Claim 1 for the preparation of a compound having the following general formula:

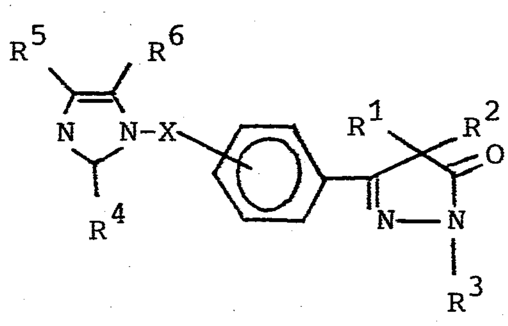

and pharmaceutically acceptable salts thereof, wherein $R^1$, $R^2$, $R^3$, $R^4$, $R^5$, $R^6$ and X are as defined in Claim 1.

3. A process according to Claim 1 for the preparation of a compound having the following general formula:

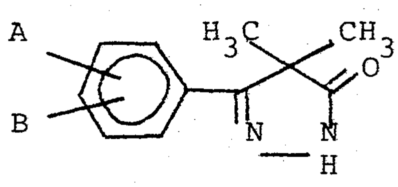

and pharmaceutically acceptable salts thereof, wherein A and B, which may be the same or different, are each an alkoxy radical of from one to six carbon atoms.

4. A process according to Claim 1 or 2 for the preparation of a compound having the following general formula:

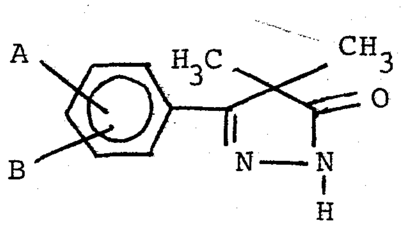

and pharmaceutically acceptable salts thereof, wherein B is a hydrogen atom and A is as defined in parts (a) to (h) and (j) above in Claim 1.

5. A process according to Claim 1, or to Claim 4 when appendant to Claim 1, wherein B is a hydrogen atom and A is one of the following: an imidazole radical; an imidazole radical substituted by one or more of the following: a $C_{1-6}$ lower alkyl radical, a $C_{1-6}$ S-lower alkyl radical, and a $-CH_2OH$ radical; a tetrahydrobenzimidazole radical; a benzimidazole radical; a 1,2,4-triazole radical; a 2-thiazoline radical; a 4-hydroxy-piperidine radical; a 1,4-dioxa-8-azaspiro[4,5]

decane radical; or a N-methylpiperazine radical.

6. A process according to Claim 5, for producing one of the following:
2,4-dihydro-5-[4-(1H-imidazol-1-yl)phenyl]-4,4-dimethyl-3H-pyrazol-3-one;
2,4-dihydro-5-[4-(1H-benzimidazol-1-yl)phenyl]-4,4-dimethyl-3H-pyrazol-3-one;
2,4-dihydro-5-[4-(4,5,6,7-tetrahydro-1H-benzimidazol-1-yl)phenyl]-4,4-dimethyl-3H-pyrazol-3-one;
2,4-dihydro-5[4-(1,4-dioxa-8-azaspiro[4,5]dec-8-yl)phenyl]-4,4-dimethyl-3H-pyrazol-3-one; and
2,4-dihydro-5-[4-(1H-1,2,4-triazol-1-yl)phenyl]-4,4-dimethyl-3H-pyrazol-3-one; or a pharmaceutically acceptable salt thereof.

7. A process according to Claim 3, for producing the compound:
2,4-dihydro-5-(3,4-dimethoxyphenyl)-4,4-dimethyl-3H-pyrazol-3-one; or a pharmaceutically acceptable salt thereof.

8. A process according to Claim 1 or 2, for producing the compound:
4-ethyl-2,4-dihydro-5-[4-(1H-imidazol-1-yl)phenyl]-4-methyl-3H-pyrazol-3-one; or a pharmaceutically acceptable salt thereof.

9. A process for producing a pharmaceutical composition, which comprises combining an effective amount of a compound of formula I as defined in Claim 1, or a salt thereof, with a pharmaceutically acceptable carrier.